# EUROPEAN PATENT SPECIFICATION

(11) **EP 2 233 478 B1**
(45) Date of publication and mention of the grant of the patent: **13.03.2013**
(21) Application number: 10005765.2
(22) Date of filing: 09.12.2005
(51) Int. Cl.: C07D 307/46, C07D 307/42

(54) **Preparation of 2,5-bis(hydroxymethyl)furan from 2,5-(hydroxymethyl)furaldehyde to using a nickel-zirconium catalyst**
Herstellung von 2,5-Bis-(hydroxymethyl)furan aus 2,5-(Hydroxymethyl)furaldehyd unter Verwendung eines Nickel-Zirkon Katalysators
Préparation de 2,5-bis(hydroxymethyl)furan à partir de 2,5-(hydroxymethyl)furaldehyde utilisant un catalyseur à base de nickel et ziconium

(30) Priority: 10.12.2004 US 635406 P; 02.03.2005 US 70063
(43) Date of publication of application: 29.09.2010
(62) Divisional of application: 05853500.6
(73) Proprietor: ARCHER-DANIELS-MIDLAND COMPANY, Decatur, IL 62525 (US)
(72) Inventor: Sanborn, Alexandra J., Lincoln IL 62656 (US)
(74) Representative: von Kreisler Selting Werner

(56) References cited:
- EP-A1- 0 862 946
- US-A- 3 083 236
- SÜD-CHEMIE AG: "Future Perspectives" ANNOUNCEMENT SUED CHEMIE, XX, XX, 1 January 2006 (2006-01-01), XP002378773

## Description

### Field Of The Invention

Improved methods of producing chemical compounds are included herein. The dehydration reaction of common carbohydrates to form commercially important compounds, furan derivatives, and methods of optimizing the reactions to efficiently synthesize the products, as well as improved methods of purification are included herein.

### Background Of The Invention

2,5-(Hydroxymethyl)furaldehyde, also known as 2,5-(hydroxymethyl)furfural (HMF), has many important industrial and commercial applications, largely due to its many functional groups and ability to serve as a precursor in many polymerization reactions. HMF, for example, is a suitable starting source for the formation of various furan monomers required for the preparation of non petroleum derived polymeric materials HFM, as well as other 2,5-disubstituted furanic derivatives, also has great potential for use in the field of intermediate chemicals from regrowing resources. Also due to its various functionalities, HMF may be used to produce a wide range of products, including, but not limited to, polymers, solvents, surfactants, pharmaceuticals, and plant protecting agents. HMF is shown in the structure below:

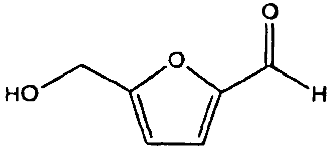

The use of HMF and other furfural derivatives may be compared with the use of corresponding benzene-based macromolecular compounds. In order to be cost-effective and compete in this market, HMF must be able to be produced at competitive prices. The production of HMF has been studied for years, but an efficient and cost-effective method of producing HMF in high yields has yet to be found. HMF is primarily produced from the dehydration reaction of a carbohydrate compound, particularly monosaccharides, including glucose and fructose. Complications arise from the rehydration of HMF after the dehydration occurs, which often yields the by-products of levulinic acid, and formic acid. Another competing side reaction is the polymerization of HMF and/or fructose to form humin polymers.

Hexoses are the preferred carbohydrate source from which HMF is formed. Fructose is the preferred hexose used for the dehydration reaction to form HMF. This is in part because fructose has been shown to be more amendable to the dehydration reaction to a form HMF. Fructose is shown by the structures below:

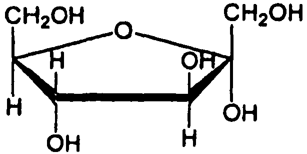

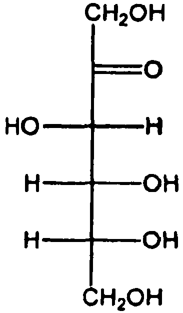

Fructose however, is more expensive than other hexoses, such as glucose (dextrose), and maltose, for example. Early processes and procedures for the production of HMF concentrated on the use of crystalline fructose, but its widespread use is prevented by its high cost. Other sources of fructose, including high-fructose corn syrup (HFCS), have been used to produce HMF and other furan derivatives. Szmant and Chundury used high fructose corn syrup as a starting material in forming HMF, as disclosed in a 1981 article in J. Chem. Tech. Biotechnol., 31, (pgs. 135-145). Szmant uses a variety of carbohydrates as starting material, but designs reaction conditions specific to each fructose source. Szmant, for example, uses a boron trifluoride catalyst (BF₃ Et₂O) with DMSO as a solvent in the conversion of HFCS to HMF, but utilizes different catalyst/solvent combinations with different starting materials. Use of BF₃ Et₂O as a catalyst is not economically practical since it cannot be recovered and re-used. Furthermore, Szmant requires the use of a Pluronic emulsifier to suppress foaming. Szmant also requires bubbling of nitrogen to suppress oxidation. Still further, Szmant requires the use of DMSO as a solvent, which is not easily separable from the HMF product, and therefore creates difficulties with product recovery. It is very desirable, therefore, to develop an industrially practicable process for producing HMF in high purity.

U.S. Patent No. 6,706,900 to Grushin et al. (Grushin '900) also discloses the dehydration of fructose in the form of high-fructose corn syrup, to form HMF as an intermediate; but this process is performed in the context of forming diformylfuran, also known as 2,5-dicarboxaldehyde (DFF). The reaction proceeds in an aqueous environment, and the HMF that is formed is not isolated from the reaction mixture, but rather is directly converted to DFF without an isolation step. The reaction conditions of Grushin '900 are therefore not constrained by considerations of product yields of HMF, as it is formed as an intermediate that is not isolated as a product. More importantly from a practical commercial standpoint, Grushin '900 is not constrained by considerations of isolating HMF from the product mixture. An efficient method for producing HMF in desirable yields and sufficiently high purity from a natural and industrially convenient fructose source that may include other mixed carbohydrates has yet to be found.

Water has in the past been used as a solvent of choice in dehydration reactions forming HMF because of the solubility of fructose in water. Aqueous conditions, however, have proven to deleteriously affect the dehydration reaction of fructose to HMF in a variety of ways. Aqueous conditions have led to decreased yield of HMF as low selectivity for the dehydration reaction has been demonstrated. Furthermore, solvation of protons in water highly reduces the catalytic activity for the dehydration reaction. Low selectivity of the dehydration reaction simultaneously leads to increased polymerization reactions and humin formation, which also interfere with the synthesis of HMF.

In an attempt to solve such problems associated with aqueous systems, one proposed solution involves an improvement by simultaneously extracting HMF after the dehydration reaction. A similar attempt to improve yields involves the adsorption of HMF on activated carbon. The key factor in these processes is a rapid removal of HMF from the acidic medium in which it is formed. However, these systems generally suffer from high dilution or partially irreversible adsorption of HMF.

In another attempt to solve the problems of aqueous systems, an organic solvent may be added to the aqueous solution, such as, for example, butanol or dioxane. Such systems, however, present a difficulty in that rehydration of HMF is common and ether formation of HMF occurs with the solvent if alcohols are employed. High yields of HMF, therefore, were not found with the addition of these organic solvents. In a further attempt to provide an adequate solvent system, aqueous solvent mixtures and anhydrous organic solvents have also been employed to ensure favorable reaction conditions. Examples of anhydrous organic solvents used include dimethylformamide, acetonitrile, dimethylsulfoxide, and polyethylene glycol.

Dimethylsulfoxide (DMSO), for example, has been extensively studied and employed as a solvent in the dehydration reaction to form HMF. Improved yields of HMF have been reached with ion exchangers or boron trifluoride etherate as a catalyst, and even without any catalyst. DMSO presents a problem, however, in that recovery of HMF from the solvent is difficult.

Furthermore, although dehydration reactions performed in solvents with high boiling points, such as dimethylsulfoxide and dimethylformamide, have produced improved yields, the use of such solvents is cost-prohibitive, and additionally poses significant health and environmental risks in their use. Still further, purification of the product via distillation has not proven effective for a variety of reasons. First of all, on long exposure to temperatures at which the desired product can be distilled, HMF and impurities associated with the synthetic mixture tend to be unstable and form tarry degradation products. Because of this heat instability, a falling film vacuum still must be used. Even in use with such an apparatus however, resinous solids form on the heating surface causing a stalling in the rotor, and the frequent shutdown resulting therefrom makes the operation inefficient.

Catalysts may also be used to promote the dehydration reaction. Some commonly used catalysts include cheap inorganic acids, such as H₂SO₄, H₃PO₄, HCl, and organic acids such as oxalic acid, levulinic acid, and p-toluene sulfonic acid. These acid catalysts are utilized in dissolved form, and as a result pose significant difficulties in their regeneration and reuse, and in their disposal. In order to avoid these problems, solid sulfonic acid catalysts have also been used. Solid acid resins, however, are limited in use by the formation of deactivating humin polymers on their surfaces under conditions taught by others. Other catalysts, such as boron trifluoride etherate, can also be used. Metals, such as Zn, Al, Cr, Ti, Th, Zr, and V can be used as ions, salts, or complexes as catalysts. Such use has not brought Improved results, however, as yields of HMF have continued to be low. Ion exchange catalysts have also been used, but have also delivered low HMF yields under conditions taught by others, and further limit the reaction temperature to under 130°C.

EP-A-0 862 946 is directed to catalysts based on zirconiumhydroxide and their use in hydrogen transfer reactions, in particular Meerwein-Ponndorf-Verley-Reduction and Oppenauer-Oxidation.

Süd-Chemie, Future Perspectives, 2006 describes hydrogenation catalysts.

### Summary of the Invention

The object of the present invention is solved on the basis of claims 1 to 13.

Provided herein is a method of preparing 2,5-bis-(hydroxymethyl)furan comprising: heating a reaction mixture comprising 2,5-(hydroxymethyl)furaldehyde, a solvent, and a catalyst system comprising nickel and zirconium at a temperature, for a time, and at a pressure sufficient to promote reduction of the 2,5-(hydroxymethyl)furaldehyde to 2,5-bis-(hydroxymethyl)furan to produce a product mixture comprising 2,5-bis-(hydroxymethyl)furan.

In an embodiment, the purification of the product isolate may be performed by a process selected from the group consisting of short path distillation, thin film evaporation, wiped film evaporation, crystallization, and adsorption to an inert adsorbent. Adsorbents include silica, carbon, alumina, and other resins. A non-volatile flowing agent may be added to the product isolate to enhance separation. The non-volatile flowing agent may be chosen from the group consisting of polyethylene glycol, polyethylene glycol monoether, polyethylene glycol diether, and combinations thereof. In a further embodiment, the non-volatile flowing agent may be purified to a re-usable form after it has performed its role in the purification process. Such purification process may take place with the use of carbon as disclosed herein.

Provided herein is a method of preparing 2,5-bis-(hydroxymethyl)furan. The method includes heating a reaction mixture comprising 2,5-(hydroxymethyl)furaldehyde, a solvent, and a catalyst system comprising nickel and zirconium at a temperature, for a time, and at a pressure sufficient to promote reduction of the 2,5-(hydroxymethyl)furaldehyde to 2,5-bis-(hydroxymethyl)furan to produce a product mixture comprising 2,5-bis-(hydroxymethyl)furan.

In one embodiment, the method provides that greater than 90% of the 2,5-(hydroxymethylfuraldehyde) is converted to 2,5-bis-(hydroxymethyf)furan. In another embodiment, greater than 95% of the 2,5-(hydroxymethylfuraldehyde) is converted to 2,5-bis-(hydroxymethyl)furan, and in yet a further embodiment, greater than 99% of the 2,5-(hydroxymethylfuraldehyde) is converted to 2,5-bis-(hydroxymethyl)furan.

In an embodiment, the method takes place with a temperature which is between about 125° C and about 175° C. In another embodiment, the method takes place with a temperature which is between about 140° C and about 160° C. In an embodiment, the pressure is between about 1,000 pounds per square inch and about 1,400 pounds per square inch. In another embodiment, the pressure is between about 1050 pounds per square inch and about 1,250 pounds per square inch.

In an embodiment, the time sufficient to promote reduction of the 2,5-(hydroxymethyl)furaldehyde to 2,5-bis-(hydroxymethyl)furan is less than about three hours. In another embodiment, the time sufficient to promote reduction of the 2,5-(hydroxymethyl)furaldehyde to 2,5-bis-(hydroxymethyl)furan is less than about two hours. In a further embodiment, the time sufficient to promote reduction of the 2,5-(hydroxymethyl)furaldehyde to 2,5-bis-(hydroxymethyl)furan is about one hour.

In an embodiment, the method of preparing 2,5-bis-(hydroxymethyl)furan further includes isolating 2,5-bis-(hydroxymethyl)furan from the product mixture by filtration to remove the catalyst and rotary evaporation to remove the solvent. In an embodiment, the solvent is one of ethyl acetate, acetate, methyl acetate, butylacetate, isopropanol, and butanol. In another embodiment, the reaction mixture comprising 2,5-(hydroxymethyl)furaldehyde is a crude reaction mixture.

The present invention provides in embodiments:
(31) A method of preparing 2,5-bis-(hydroxymethyl)furan comprising: heating a reaction mixture comprising 2,5-(hydroxymethyl)furaldehyde, a solvent, and a catalyst system comprising nickel and zirconium at a temperature, for a time, and at a pressure sufficient to promote reduction of the 2,5- (hydroxymethyl)furaldehyde to 2,5-bis-(hydroxymethyl)furan to produce a product mixture comprising 2,5-bis-(hydroxymethyl)furan.
(32) The method of embodiment (31) wherein greater than 90% of the 2,5-(hydroxymethylfuraldehyde) is converted to 2,5-bis-(hydroxymethyl)furan.
(33) The method of embodiment (31) wherein the temperature is between about 125°C and about 175°C.
(34) The method of embodiment (31) wherein the pressure is between about 1000 and about 1500 pounds per square inch.
(35) The method of embodiment (31) wherein the time sufficient to promote reduction of the 2,5-(hydroxymethyl)furaldehyde to 2,5-bis-(hydroxymethyl)furan is less than about three hours.
(36) The method of embodiment (31) wherein the solvent is one of ethyl acetate, acetate, methyl acetate, butyl acetate, isopropanol, and butanol.

### Detailed Description of the Invention

Reusable or recyclable catalysts are preferred for use in the reaction, as they provide for increased efficiency, and economic and industrial feasibility. As used herein, the term "recyclable catalyst" refers to a catalyst which is not irreversibly expended as a result of the reaction. In other words, the catalyst may be used again. Examples of recyclable or reusable catalysts include solid acid catalysts, ion-exchange resins, zeolites, Lewis acids, clays, and molecular sieves. Solid acid catalysts often comprise a solid material which has been functionalize to impart acid groups that are catalytically active. Solid acid catalysts may have a broad range of composition, porosity, density, type of add groups and distribution of acid groups. Solid acid catalysts may be recovered and reused, optionally with a treatment to regenerate any activity that may have been lost in use. Some solid acid catalysts that may be used in the disclosed process include, but are not limited to Amberlyst 35, Amberlyst 36, Amberlyst 15, Amberlyst 131 (Rohm and Haas, Woodridge, IL), Lewatit S2328, Lewatit K2431, Lewatit S2568, Lewatit K2629 (Sybron Corp, Birmingham, NJ), Dianion SK104, Dianion PK228, Dianion RCP160, RCP21H, Relite RAD/F (Mitsubishi Chemical, White Plains, NY), and Dowex 50WX4 (Dow Chemical).

One example of a solvent that may be used is a polar solvent. The polar solvent maybe a polar aprotic solvent. Examples of possible solvents Include, but are not limited to, 1-methyl-2-pyrrolidinone, dimethylacetamide, dimethylformamide, dimethyl sulfoxide, methyl ethyl ketone, methyl isobutylketone, acetonitrile, propionitrile, and combinations thereof.

By removing water as it is formed, side-reactions are thereby minimized, and an increased yield has been observed. Water removal may take place via evaporation. A rotary evaporation machine may be employed to promote water removal. The use of a rotary evaporator, or "rotovap," is well-known in the art. Water removal may also be carried out by evaporation from the reaction mixture and condensation as ice or water on a cold finger or reflux condenser. Water may also be removed by distillation, including azeotropic distillation with a water-entraining solvent which may optionally be stripped of water and the water depleted solvent returned to the reaction vessel. A suitable distillation apparatus, such as a Barrett type receiver may also be employed. A water-absorbing material may also be used to remove water. Such materials are well-known in the art, and include molecular sieves.

In one embodiment, the reactions disclosed herein are performed at moderately high temperatures, typically In a range of from about 95° to about 125°C. In a further embodiment, the temperature range is from about 105° C to about 115° C. It is preferable to use temperatures below 200 degrees Celsius. The reactions disclosed herein typically occur in a time frame of from about one to about six hours. More typically, the reactions take from about two hours to about five and a half hours.

As used herein, the term "zeolite" refers to a hydrated silicate of aluminum and one or both of sodium and calcium. Examples include analcite, chabazite, heulandite, natrolite, stilbite, thomsonite, in either powder or pellet form. Commercial zeolites products include CBV 3024 and CBV 5534G (Zeolyst International), T-2665, T-4480 (United Catalysis, Inc), LZY 64 (Union Carbide), and H-ZSM-5 (PQ Corporation).

As used herein, Cornsweet 90 refers to a high fructose corn syrup product of commerce nominally containing 60% to 70% fructose. High fructose corn syrup refinery intermediate and by-product is a fructose-rich stream generated in a fractionation system positioned after an isomerization column in the production of high fructose corn syrup. A suitable process stream from crystallizing fructose is called "mother liquor" and comprises a solution of fructose in ethanol. Typically this process stream is about 24% solids, almost all of the solids being fructose, and contains about 60% ethanol. A similar mother liquor from glucose crystallization contains about 50% solids. Mixed carbohydrate sources can be obtained by blending carbohydrates, such as by adding crystalline fructose to high fructose corn syrup.

As used herein, "reaction yield" is calculated using the equation (moles of product/moles of starting material)" 100. Product purity is reported on a weight percent basis.

As used in this equation, "starting material" refers to the fructose present in the carbohydrate source, mixed carbohydrate source, or other reactant for the particular dehydration reaction.

As used herein, the term "fructose **source"** refers to a material that comprises sucrose. Typical embodiments are solutions having at least 25% sucrose by solute weight, and which may include other materials such as other carbohydrate compounds. Preferably, the carbohydrate compounds are hexoses. The versatility of the reaction conditions provided herein allow an industrially convenient source to be used as the starting material, that is to say, the reaction is not limited to a particular carbohydrate source or to fructose of high purity.

Suitable fructose sources typically include high fructose corn syrup (HFCS) or any HFCS refining process stream that includes at least 25% sucrose. HFCS is typically commercially available in products comprising solutions having 42% to 95% fructose by solute weight which are typically sold for use as industrial scale sweeteners. The most economical embodiments of the invention use HFCS having about 90% sucrose by solute weight. However, less economical embodiment's invention can be practiced with sources having less sucrose by weight. To improve economic efficiencies, less pure sucrose sources can be conveniently blended with higher purity sucrose sources or even crystalline sucrose to achieve a solution having at least 25% sucrose by solute weight.

Optional neutralization of the product isolate is carried out by addition of a suitable alkali substance, such as a basic ion exchange resin, potassium hydroxide, or sodium hydroxide. This neutralization step allows for subsequent product recovery by distillation without heat-catalyzed degradation or polymerization, resulting in the elimination of tarry degradation products and resinous solids being formed in distillation. This neutralization step also allows for subsequent product recovery with a flowing agent without heat-catalyzed degradation or polymerization, resulting in the elimination of tarry degradation products and resinous solids being formed in distillation.

Purity was determined by ¹³C NMR and Proton NMR, in some cases by capillary GC, and in some cases by UV adsorption.

As used herein, the term "non-aqueous mixture" refers to a mixture comprising a non-aqueous solvent and at least one other component, wherein the content of the solvent is greater than the content of the at least one other component, as measured by volume. The at least one other component may comprise, without limitation, a water-containing substrate, such as HFCS, or an organic solvent. Non-aqueous solvents are usually measured by volume, and other components are usually measured by weight.

As used herein, the term "isolate" refers to the process of preservation of a material originally present in a product mixture after the product mixture has been subjected to a step to remove other material from the product mixture, as well as the isolated material resulting from the process. Examples of "other material" that is removed includes solid material, such as catalyst by methods including the processes of filtration, decantation, centrifugation, and washing. Filtration may be performed by one of the processes selected from the group comprising to gravity filtration, vacuum filtration, and suction filtration.

The term "non-volatile flowing agent" as used herein refers to an inert material which, when added to a product mixture, aids in the recovery of the desired compound by distillation. In certain embodiments, the fugacity of the flowing agent is sufficiently low so that it will not volatilize as the target product is removed by evaporation.

A method of preparing 2,5-bis-(hydroxymethyl)furan is disclosed. The method includes heating a reaction mixture comprising 2,5-(hydroxymethyl)furaldehyde, a solvent, and a catalyst system comprising nickel and zirconium at a temperature, for a time, and at a pressure sufficient to promote reduction of the 2,5-(hydroxymethyl)furaldehyde to 2,5-bis-(hydroxymethyl)furan to produce a product mixture comprising 2,5-bis-(hydroxymethyl)furan. In an embodiment, the reaction mixture comprising 2,5-(hydroxymethyl)furaldehyde is a crude reaction mixture.

As used herein, the term "crude reaction mixture" refers to an unrefined or unpurified composition.

### Examples

The following are examples of the dehydration of a fructose source to a furan derivative or organic acid, as well as isolation and/or purification techniques to optimize product recovery of increased product yield.

### EXAMPLE 1

### PREPARATION OF 2,5-BIS-(HYDROXYMETHYL)FURAN (FDM) FROM CRUDE HMF REACTION MIXTURE

The sample of HMF material (30.01 g, 66% HMF) was placed in a 1L Parr reactor vessel with ethyl acetate (350 mL) and 2.0 g of G-69B. G-69B is a powdered catalyst obtained from Sud-Chemie, Louisville, Kentucky, containing nominally 62% Nickel on Kieselguhr, with a Zirconium promoter, and has an average particle size of 10-14 microns. The vessel was purged 3 X 500 psi hydrogen with vigorous stirring (1000 rpm). The pressure was then maintained at 1250-1050 psi with heating to 150°C for 1 hour. The reaction was allowed to cool and the catalyst removed by filtration. The solvent was removed by rotary evaporation to provide 27.32 g of brown liquid that solidified on cooling. TLC analysis indicated the complete conversion of HMF to FDM. ¹H NMR data reveal a high purity product (>90%). The overall yield of FDM from HMF is 100%. GC/MS data revealed complete conversion of HMF to FDM m/z = 128, 111, 97.

### EXAMPLE 2

### PREPARATION OF 2,5-BIS-(HYDROXYMETHYL)FURAN (FDM) FROM CRUDE HMF REACTION MIXTURE

The sample of HMF material (46.09 g, 45% HMF) was placed in a 1 L Parr reactor vessel with ethyl acetate (350 mL) and 6.15 g of G-69B. The vessel was purged 3 x 500 psi hydrogen with vigorous stirring (1000 rpm). The pressure was then maintained at 1350 psi with heating to 150°C for 1 hour. The reaction was allowed to cool and the catalyst removed by filtration. The solvent was removed by rotary evaporation to provide 18.48 g of brown solid. ¹H NMR and gc/ms data reveal a high purity product (>95%). The overall yield of FDM from HMF is 90%. NMR (δ, 1H): 4.54 (s, 2.0 H); 6.20 (s, 1.0 H).

## Claims

1. A method of preparing a 2,5-bis-(hydroxymethyl)furan comprising:
heating a reaction mixture comprising 2,5-(hydroxymethyl)furaldehyde, a solvent, and a catalyst system consisting of nickel and a zirconium promoter on a substrate at a temperature, for a time, and at a pressure sufficient to promote reduction of the 2,5-(hydroxymethyl)furaldehyde to 2,5-bis-(hydroxymethyl)furan to produce a product mixture comprising 2,5-bis-(hydroxymethyl)furan.

2. The method of claim 1, wherein greater than 90% of the 2,5-(hydroxymethylfuraldehyde) is converted to 2,5-bis-(hydroxymethyl)furan.

3. The method of claim 1, wherein the temperature is between about 125°C to about 175°C.

4. The method of claim 1, wherein the pressure is between about 1000 to about 1500 pounds per square inch.

5. The method of claim 1, wherein a time sufficient to promote reduction of the 2,5-(hydroxymethyl)furaldehyde to 2,5-bis-(hydroxymethyl)furan is less than about three hours.

6. The method of claim 1, wherein the solvent is one of ethyl acetate, acetate, methyl acetate, butyl acetate, isopropanol, and butanol.

7. The method of claim 1, wherein the reaction mixture comprising the 2,5-(hydroxymethyl)furaldehyde is a crude reaction mixture.

8. The method of claim 1, wherein greater than 95% of the 2,5-(hydroxymethylfuraldehyde) is converted to 2,5-bis-(hydroxymethyl)furan.

9. The method of claim 1, wherein a time sufficient to promote reduction of the 2,5-(hydroxymethyl)furaldehyde to 2,5-bis-(hydroxymethyl)furan is about one hour.

10. The method of claim 1, wherein the substrate is Kieselguhr.

11. The method of claim 1, wherein the catalyst system consists of nominally 62% nickel and a zirconium promoter.

12. The method of claim 1, wherein the catalyst system consists of nominally 62% nickel and a zirconium promoter on a substrate.

13. The method of claim 12, wherein the substrate is Kieselguhr.

## Patentansprüche

1. Verfahren zur Herstellung eines 2,5-Bis(hydroxymethyl)furans, umfassend:
Erhitzen eines Reaktionsgemischs, das 2,5-(Hydroxymethyl)furaldehyd, ein Lösungsmittel und ein Katalysatorsystem, das aus Nickel und einem Zirconium-Promotor auf einem Substrat besteht, umfasst, bei einer Temperatur, während einer Zeit und unter einem Druck, die ausreichen, um die Reduktion des 2,5-(Hydroxymethyl)furaldehyds zu 2,5-Bis(hydroxymethyl)furan zu fördern, wobei ein Produktgemisch entsteht, das 2,5-Bis(hydroxymethyl)furan umfasst.

2. Verfahren gemäß Anspruch 1, wobei mehr als 90% des 2,5-(Hydroxymethyl)furaldehyds zu 2,5-Bis(hydroxymethyl)furan umgesetzt werden.

3. Verfahren gemäß Anspruch 1, wobei die Temperatur zwischen etwa 125 °C und etwa 175 °C liegt.

4. Verfahren gemäß Anspruch 1, wobei der Druck zwischen etwa 1000 und etwa 1500 psi (pounds per square inch) liegt.

5. Verfahren gemäß Anspruch 1, wobei die zur Förderung der Reduktion des 2,5-(Hydroxymethyl)furaldehyds zu 2,5-Bis(hydroxymethyl)furan ausreichende Zeit weniger als etwa drei Stunden beträgt.

6. Verfahren gemäß Anspruch 1, wobei das Lösungsmittel aus Ethylacetat, Acetat, Methylacetat, Butylacetat, Isopropanol und Butanol ausgewählt ist.

7. Verfahren gemäß Anspruch 1, wobei das Reaktionsgemisch, das das 2,5-(Hydroxymethyl)furaldehyd umfasst, ein rohes Reaktionsgemisch ist.

8. Verfahren gemäß Anspruch 1, wobei mehr als 95% des 2,5-(Hydroxymethyl)furaldehyds zu 2,5-Bis(hydroxymethyl)furan umgesetzt werden.

9. Verfahren gemäß Anspruch 1, wobei die zur Förderung der Reduktion des 2,5-(Hydroxymethyl)furaldehyds zu 2,5-Bis(hydroxymethyl)furan ausreichende Zeit etwa eine Stunde beträgt.

10. Verfahren gemäß Anspruch 1, wobei es sich bei dem Substrat um Kieselgur handelt.

11. Verfahren gemäß Anspruch 1, wobei das Katalysatorsystem aus nominell 62% Nickel und einem Zirconiumpromotor besteht.

12. Verfahren gemäß Anspruch 1, wobei das Katalysatorsystem aus nominell 62% Nickel und einem Zirconiumpromotor auf einem Substrat besteht.

13. Verfahren gemäß Anspruch 12, wobei es sich bei dem Substrat um Kieselgur handelt.

## Revendications

1. Procédé de préparation d'un 2,5-bis-(hydroxyméthyl)furane comprenant l'étape consistant à :
chauffer un mélange réactionnel comprenant du 2,5-(hydroxyméthyl)furaldéhyde, un solvant et un système catalytique consistant en du nickel et un promoteur de zirconium sur un substrat à une température, pendant un temps et à une pression suffisants pour favoriser la réduction du 2,5-(hydroxyméthyl)furaldéhyde en 2,5-bis-(hydroxyméthyl)furane pour produire un mélange de produits comprenant du 2,5-bis-(hydroxyméthyl)furane.

2. Procédé selon la revendication 1, dans lequel plus de 90 % du 2,5-(hydroxyméthyl)furaldéhyde sont convertis en 2,5-bis-(hydroxyméthyl)furane.

3. Procédé selon la revendication 1, dans lequel la température est entre environ 125 °C à environ 175 °C.

4. Procédé selon la revendication 1, dans lequel la pression est entre environ 1 000 à environ 1 500 livres par pouce carré.

5. Procédé selon la revendication 1, dans lequel un temps suffisant pour favoriser la réduction du 2,5-(hydroxyméthyl)furaldéhyde en 2,5-bis-(hydroxyméthyl)furane est de moins d'environ trois heures.

6. Procédé selon la revendication 1, dans lequel le solvant est l'un parmi l'acétate d'éthyle, l'acétate, l'acétate de méthyle, l'acétate de butyle, l'isopropanol et le butanol.

7. Procédé selon la revendication 1, dans lequel le mélange de réaction comprenant le 2,5-(hydroxyméthyl)furaldéhyde est un mélange de réaction brut.

8. Procédé selon la revendication 1, dans lequel plus de 95 % du 2,5-(hydroxyméthyl)furaldéhyde sont convertis en 2,5-bis-(hydroxyméthyl)furane.

9. Procédé selon la revendication 1, dans lequel un temps suffisant pour favoriser la réduction du 2,5-(hydroxyméthyl)furaldéhyde en 2,5-bis-(hydroxyméthyl)furane est d'environ une heure.

10. Procédé selon la revendication 1, dans lequel le substrat est du Kieselguhr.

11. Procédé selon la revendication 1, dans lequel le système catalytique consiste nominalement en 62 % de nickel et un promoteur de zirconium.

12. Procédé selon la revendication 1, dans lequel le système catalytique consiste nominalement en 62 % de nickel et un promoteur de zirconium sur un substrat.

13. Procédé selon la revendication 12, dans lequel le substrat est du Kieselguhr.
